(19) Europäisches Patentamt
European Patent Office
Office européen des brevets

(11) EP 1 375 562 A1

(12) **EUROPEAN PATENT APPLICATION**
published in accordance with Art. 158(3) EPC

(43) Date of publication:
**02.01.2004 Bulletin 2004/01**

(21) Application number: **01271889.6**

(22) Date of filing: **26.12.2001**

(51) Int Cl.⁷: **C08G 73/10**, G02F 1/1337, C09K 19/56, C07C 217/86, C07C 229/60

(86) International application number:
**PCT/JP2001/011487**

(87) International publication number:
**WO 2002/051908 (04.07.2002 Gazette 2002/27)**

(84) Designated Contracting States:
**AT BE CH CY DE DK ES FI FR GB GR IE IT LI LU MC NL PT SE TR**

(30) Priority: **26.12.2000 JP 2000394140**

(71) Applicant: **Nissan Chemical Industries, Ltd.**
**Chiyoda-ku, Tokyo 101-0054 (JP)**

(72) Inventors:
- **HOSAKA, Kazuyoshi**
**Nissan Chemical Industries, Ltd.**
**Funabashi-shi, Chiba 274-8507 (JP)**
- **NAWATA, Hideyuki**
**Nissan Chemical Industries, Ltd.**
**Funabashi-shi, Chiba 274-8507 (JP)**
- **NIHIRA, Takayasu**
**Nissan Chemical Industries, Ltd.**
**Funabashi-shi, Chiba 274-8507 (JP)**

(74) Representative:
**Wächtershäuser, Günter, Prof. Dr. et al**
**Wächtershäuser & Hartz**
**Patentanwälte**
**Weinstrasse 8**
**80333 München (DE)**

(54) **DIAMINOBENZENE DERIVATIVES, POLYIMIDE PRECURSORS AND POLYIMIDES PREPARED BY USING THA SAME, AND LIQUID CRYSTAL ALIGNING AGENTS**

(57) A diaminobenzene derivative represented by the formula (1):

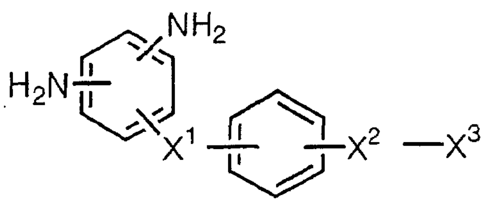

(1)

wherein $X^1$ is a bivalent organic group selected from $-OCH_2-$, $-CH_2O-$, $-COOCH_2-$ and $-CH_2OOC-$, $X^2$ is -O-, and $X^3$ is an alkyl or fluoroalkyl group having from 1 to 22 carbon atoms, or a cyclic substituent selected from aromatic rings, aliphatic rings, heterocyclic rings and their substituted groups; a polyimide precursor and a polyimide synthesized by using the diaminobenzene derivative as a part of the material; and a treating agent for liquid crystal alignment containing the polyimide precursor and/or the polyimide.

EP 1 375 562 A1

## Description

### TECHNICAL FIELD

The present invention relates to a novel diaminobenzene derivative, a polyimide precursor and a polyimide synthesized by using the compound as a part of the material, and a treating agent for liquid crystal alignment containing such a polymer.

### BACKGROUND ART

Heretofore, a polyimide has been widely used as a protective material or an insulating material in the electric or electronic field by virtue of its characteristics such as high mechanical strength, heat resistance and solvent resistance. Especially in an application to an alignment film for a liquid crystal display device, a polyimide has been employed because of the uniformity and durability of the coating film surface. However, along with attempts for high densification and high performance of liquid crystal displays, the surface properties of the polyimide coating film have become increasingly important, and it is now required to impart new properties which conventional polyimides do not have.

In recent years, developments in the electric and electronic fields have been remarkable, and higher properties have been correspondingly required for the material to be employed. Under these circumstances, also for a liquid crystal alignment film, it has been required to impart new properties which conventional polyimides do not have, in attempts for high densification and high performance of the displays.

As a means to obtain a high pretilt angle required for a polyimide liquid crystal alignment film, JP-A-64-25126 and JP-A-5-27244 propose a treating agent for liquid crystal alignment comprising a polyimide precursor or a polyimide containing a side chain, prepared by using, as a material, a diamine having a long chain alkyl group, etc. However, the diamine proposed in these references, has had a problem with respect to the efficiency to increase the pretilt angle relative to the amount introduced, or with respect to the thermal stability of the pretilt angle.

when properties other than the pretilt angle, are also to be imparted to the liquid crystal alignment film, the amount of the diamine introduced to increase the pretilt angle should better be small, as the degree of freedom in polymer design will thereby be broadened. However, if the amount the diamine introduced, is too small, it becomes impossible to secure the required pretilt angle.

Further, with a liquid crystal alignment film of the type wherein an alkyl group is introduced into a polyimide, the thermal stability of the pretilt angle has been inadequate. Namely, a conventional polyimide alignment film having an alkyl group introduced, has had a problem such that the pretilt angle decreases when heated at a temperature higher than the isotropic temperature of liquid crystal (hereinafter referred to as isotropic treatment), although the pretilt angle after injection of liquid crystal can be increased. The decrease in the pretilt angle by isotropic treatment becomes particularly remarkable in a case where the pretilt angle is high or in a case where the curing temperature at the time of forming the liquid crystal alignment film, is low. Further, at the time of forming a polyimide film on a substrate, it is common to carry out baking at a high temperature of from 200 to 300°C, and there has been a problem such that as the heat resistance of the alkyl group side chain itself is insufficient, the pretilt angle tends to decrease or fluctuate especially by high temperature baking.

In view of the above problems, the present invention is to provide a novel diaminobenzene derivative which is highly effective to increase the pretilt angle and excellent in the thermal stability of the pretilt angle, a polyimide precursor or a polyimide synthesized by using such a diaminobenzene derivative as a part of the material, and a treating agent for liquid crystal alignment containing such a polymer.

### DISCLOSURE OF THE INVENTION

As a result of an extensive study on the above-mentioned problems, the present inventors have found a diaminobenzene derivative having a specific structure, a polyimide precursor or a polyimide synthesized by using such a diaminobenzene derivative as a part of the material, and a treating agent for liquid crystal alignment containing such a polymer.

Namely, the present invention relates to a diaminobenzene derivative represented by the formula (1):

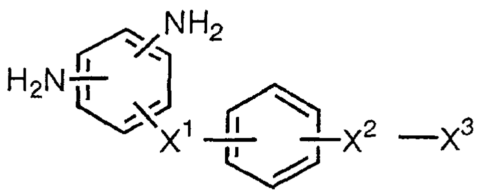

(1)

wherein $X^1$ is a bivalent organic group selected from - $OCH_2$-, -$CH_2O$-, -$COOCH_2$- and -$CH_2OOC$-, $X^2$ is -O-, and $X^3$ is an alkyl or fluoroalkyl group having from 1 to 22 carbon atoms, or a cyclic substituent selected from aromatic rings, aliphatic rings, heterocyclic rings and their substituted groups; a polyimide precursor and a polyimide synthesized by using such a diaminobenzene derivative as a part of the material; and a treating agent for liquid crystal alignment containing such a polymer.

## BEST MODE FOR CARRYING OUT THE INVENTION

Now, the present invention will be described in detail.
In the formula (1), $X^1$ is a bivalent organic group selected from -$OCH_2$-, -$CH_2O$- -$COOCH_2$- and -$CH_2OOC$-, but it is preferably -$OCH_2$-, -$COOCH_2$- or -$CH_2OOC$-, which can be readily synthesized. Further, the bonding position of $X^1$ may be any position selected from 2,3-positions, 2;4-positions, 2,5-positions, 2,6-positions, 3,4-positions or 3,5-positions to the amino groups, but preferably 2,4-positions or 3,5-positions in view of the effect to increase nucleophilic properties of the amino groups or synthetic efficiency.
In the formula (1), $X^3$ is an alkyl or fluoroalkyl group having from 1 to 22 carbon atoms, or a cyclic substituent selected from aromatic rings, aliphatic rings, heterocyclic rings and their substituted groups, preferably an alkyl group having from 6 to 22 carbon atoms or a fluoroalkyl group having from 1 to 22 carbon atoms. Further, the bonding position of $X^2$ may be any position selected from o-position, m-position or p-position to $X^1$, preferably m-position or p-position.
As described in the foregoing, the novel diaminobenzene derivative of the present invention is characterized in the structure wherein, in order to increase the effect to increase the pretilt angle, the bonding position of the side chain substituent is distanced from the sites of the amino groups, and the side chain substituent density will not be reduced when formed into a polyimide precursor or a polyimide, and further characterized in that in order to improve the thermal stability of the pretilt angle, a phenyl ring is introduced to the side chain bonding site.
The diaminobenzene derivative of the present invention can easily be synthesized and is useful as a material for a polyimide precursor and a polyimide, etc. Further, the polyimide precursor and the polyimide synthesized by using it as a part of the material, are excellent in the effect to increase the pretilt angle of liquid crystal and provide good thermal stability of the pretilt angle, and thus are useful for use as a treating agent for liquid crystal alignment.

### Synthesis of the diaminobenzene derivative

The method for synthesis of the diaminobenzene derivative of the formula (1) of the present invention is not particularly limited. For example, it can be synthesized by the following method.
A method which comprises synthesizing a dinitro compound of the formula (2) corresponding to the diaminobenzene derivative represented by the formula (1) of the present invention:

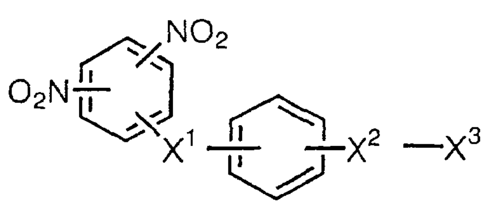

(2)

and further reducing the nitro groups by a usual method to convert them into amino groups. The reduction of nitro groups may be carried out by e.g. hydrogen gas, hydrazine or hydrogen chloride in a solvent such as ethyl acetate, toluene, tetrahydrofuran, dioxane or an alcohol by using, as a catalyst, palladium-carbon, platinum oxide Raney Nickel, platinum black, rhodium-alumina, platinum sulfide-carbon or the like.
The dinitro compound of the formula (2) can be synthesized by bonding a substituent $X^3$ via a connecting portion $X^2$ to a phenyl ring and then bonding the dinitro portion thereto via a connecting portion $X^1$.
The connecting portion $X^1$ is a bonding group such as an ether methylene bond (-$OCH_2$-), a methylene ether

bond ($-CH_2O-$) an ester methylene bond ($-COOCH_2-$) or a methylene ester bond ($-CH_2COO-$). Such a bonding group may be formed by a usual organic synthetic method. Specifically, it is common that in the case of the ether methylene bond, the corresponding dinitro group-containing halogen derivative is reacted with a hydroxyl group-substituted benzene derivative containing the connecting portion $X^2$ and the substituent $X^3$, in the presence of an alkali, or in the case of the methylene ether bond, the corresponding dinitro group-containing halogen derivative is reacted with a hydroxyl group-substituted benzene derivative containing the connecting portion $X^2$ and the substituent $X^3$, in the presence of an alkali, or in the case of the ester methylene bond, the corresponding dinitro group-containing acid chloride is reacted with a hydroxyl group-substituted benzene derivative containing the connecting portion $X^2$ and the substituent $X^3$, in the presence of an alkali.

In the case of the methylene ester bond, it is common to employ a method wherein the corresponding dinitro group-containing halogen derivative is reacted with a carboxylic acid-substituted benzene derivative containing the connecting portion $X^2$ and the substituent $X^3$, in the presence of an alkali, or the corresponding dinitro group-containing hydroxyl group-substituted derivative is reacted with an acid chloride benzene derivative having the connecting portion $X^2$ and the substituent $X^3$, in the presence of an alkali.

The connecting portion $X^1$ can be selected from the above-mentioned bonding groups, but it is preferably an ether methylene bond ($-OCH_2-$), an ester methylene bond ($-COOCH_2-$) or a methylene ester bond ($-CH_2COO-$) which can easily be synthesized.

As the above-mentioned dinitro group-containing halogen derivative, the dinitro group-containing acid chloride and the dinitro group-containing hydroxyl group-substituted derivative, 3,5-dinitrochlorobenzene, 2,4-dinitrochlorobenzene, 2,4-dinitrofluorobenzene, 3,5-dinitrobenzoic acid chloride, 2,4-dinitrobenzoic acid chloride, 3,5-dinitrobenzylchloride, 2,4-dinitrobenzylchloride, 3,5-dinitrobenzyl alcohol, 2,4-dinitrobenzyl alcohol, 2,4-dinitrophenol, 2,5-dinitrophenol, 2,6-dinitrophenol, 2,4-dinitrobenzoic acid, and 3,5-dinitrobenzoic acid, may, for example, be mentioned. A combination of these may suitably be selected depending upon the particularly purpose in view of the reaction or the availability of the material. It should be mentioned that the compounds mentioned here are merely exemplary.

The bonding position of the connecting portion $X^1$ may be at any position selected from 2,3-positions, 2,4-positions, 2,5-positions, 2,6-positions, 3,4-positions and 3,5-positions to the amino groups, but preferably 2,4-positions or 3,5-positions, in view of the effect to increase the nucleophilic property of the amino groups or synthetic efficiency.

The hydroxyl group-substituted benzene derivative containing the connecting portion $x^2$ and the substituent $X^3$ can be obtained by a usual organic synthetic method.

Specifically, it is common to employ a method of reducing a methyl benzoate group by means of $LiAlH_4$, or reacting a benzaldehyde group with formaldehyde in the presence of an alkali.

Further, the carboxylic acid-substituted benzene derivative containing the connecting portion $X^2$ and the substituent $X^3$, may also be obtained by a usual organic synthetic method. Specifically, it is common to employ a method of hydrolyzing a methyl benzoate group. Further, for the acid chloride benzene derivative, it is common to employ a method of reacting the carboxylic acid-substituted benzene derivative thus obtained in the presence of an organic acid.

The bonding position of the connecting portion $X^2$ may be at any position selected from o-position, m-position and p-position to $X^1$, but it is preferably m-position or p-position whereby the steric hindrance of the substituent $X^3$ to the amino groups will be small.

The connecting portion $X^2$ is an ether bond (-O-), and this bonding group can be formed by a usual organic synthetic method.

Specifically, it is common to employ a method of reacting a hydroxyl group-substituted benzene derivative with a halogen derivative containing the corresponding substituent $X^3$ in the presence of an alkali, or a method of reacting a halogen-containing benzene derivative with an alcohol derivative containing the corresponding substituent $X^3$ in the presence of an alkali.

The above-described substituent $X^3$ is an alkyl or fluoroalkyl group having from 1 to 22 carbon atoms, preferably an alkyl group having at least 6 carbon atoms or a fluoroalkyl group having at least one carbon atom, substantially an alkyl group having from 6 to 22 carbon atoms or a fluoroalkyl group having from 1 or 22 carbon atoms, from the viewpoint of efficiency in the synthesis.

The larger the carbon number, the higher the effect to increase the water repellency of the polyimide precursor and the polyimide, and further, when it is used for an application to a liquid crystal alignment film, the effect to increase the pretilt angle will be higher.

Further, the substituent $X^3$ may be a cyclic substituent selected from aromatic rings, aliphatic rings, heterocyclic rings and their substituted groups.

Specific examples include a benzene ring, a heterocyclic ring, a cyclohexane ring, a biphenyl ring, a terphenyl ring, a bicyclohexyl ring, a tercyclohexyl ring, a phenylcyclohexyl ring, a phenylpyridine ring, a cyclohexylpyridine ring, a phenyldioxane ring, a phenylpyrimidine ring, a cyclohexylpyrimidine ring, a phenylpyrazine ring, a cyclohexylpyrazine ring, and further one having these cyclic compounds bonded via a connecting portion such as ethylene, acetylene, an ester, oxymethylene, azo, azoxy or azomethine. It is particularly preferred to employ a benzene ring, a cyclohexane

ring, a biphenyl ring, a bicyclohexyl ring or a phenylcyclohexyl ring from the viewpoint of availability of the material or efficiency in the synthetic reaction.

Further, it is common that these cyclic compounds are substituted by various terminal groups. As such terminal groups, an alkyl group, an alkoxy group, a fluoroalkyl group, a fluoroalkoxy group, a halogen atom, a nitro group, an amino group, a cyano group, an azo group, a formyl group, an acetyl group and an acetoxy group are, for example, known. From the viewpoint of availability of the material, efficiency in the synthetic reaction and an ability to effectively provide a pretilt angle, particularly preferred is a substituted cyclic group which is substituted by a substituent selected from an alkyl group, an alkoxy group, a fluoroalkyl group and a fluoroalkoxy group.

Such a substituted cyclic group may be suitably selected to increase the heat resistance, water repellency, etc. of the polyimide precursor and the polyimide.

Polyimide precursor and polyimide

The diaminobenzene derivative of the present invention represented by the formula (1) may be subjected to polycondensation with a tetracarboxylic acid or its derivative, such as a tetracarboxylic acid, a tetracarboxylic acid dihalide or a tetracarboxylic dianhydride to synthesize a polyimide precursor or a polyimide having a specific structure in the side chain.

The method for obtaining the polyimide precursor and the polyimide of the present invention is not particularly limited. As mentioned above, the polyimide precursor is obtained by the polycondensation of the diaminobenzene derivative with a tetracarboxylic acid or its derivative, and the polyimide can be obtained by ring closure of this polyimide precursor.

The tetracarboxylic acid and its derivative to be used to obtain the polyimide precursor and the polyimide of the present invention, are not particularly limited. Their specific examples include aromatic tetracarboxylic acids such as pyromellitic acid, 2,3,6,7-naphthalene tetracarboxylic acid, 1,2,5,6-naphthalene tetracarboxylic acid, 1,4,5,8-naphthalene tetracarboxylic acid, 2,3,6,7-anthracene tetracarboxylic acid, 1,2,5,6-anthracene tetracarboxylic acid, 3,3',4,4'-biphenyl tetracarboxylic acid, 2,3,3',4-biphenyl tetracarboxylic acid, bis(3,4-dicarboxyphenyl)ether, 3,3',4,4'-benzophenone tetracarboxylic acid, bis(3,4-dicarboxyphenyl)sulfone; bis(3,4-dicarboxyphenyl)methane, 2,2-bis(3,4-dicarboxyphenyl)propane, 1,1,1,3,3,3-hexafluoro-2,2-bis(3,4-dicarboxyphenyl)propane, bis(3,4-dicarboxyphenyl)dimethylsilane, bis(3,4-dicarboxyphenyl)diphenylsilane, 2,3,4,5-pyridine tetracarboxylic acid and 2,6-bis(3,4-dicarboxyphenyl) pyridine, and their dianhydrides and their dicarboxylic diacid halides; alicyclic tetracarboxylic acids such as 1,2,3,4-cyclobutane tetracarboxylic acid, 1,2,3,4-cyclopentane tetracarboxylic acid, 1,2,4,5-cyclohexane tetracarboxylic acid, 2,3,5-tricarboxy cyclopentylacetic acid, 3,4-dicarboxy-1,2,3,4-tetrahydro-1-naphthalene succinic acid, bicyclo[3,3,0]-octane-tetracarboxylic acid and 3,5,6-tricarboxynorbornane, and their dianhydrides and their dicarboxylic diacid halides; and aliphatic tetracarboxylic acids such as 1,2,3,4-butane tetracarboxylic acid, and their dianhydrides and their dicarboxylic diacid halides. These tetracarboxylic acids and their derivatives may be used alone or in combination as a mixture of two or more of them.

Further, a polyimide obtainable by using an alicyclic tetracarboxylic acid or an aliphatic tetracarboxylic acid, or a dianhydride or dicarboxylic diacid halide thereof as a part or whole of the entire tetracarboxylic acid or its derivative, can be made to be a solvent-soluble polyimide. Particularly, polyimides obtainable by using 2,3,5-tricarboxy cyclopentyl acetic acid, 3,4-dicarboxy-1,2,3,4-tetrahydro-1-naphthalene succinic acid, bicyclo[3,3,0]-octane-tetracarboxylic acid, 1,2,3,4-butane tetracarboxylic acid, and their dianhydrides and their dicarboxylic diacid halides, show good solubility in organic polar solvents such as N-methyl pyrrolidone, N,N'-dimethyylacetoamide, N,N'-dimethylformamide and γ-butyrolactone.

In a case where the polyimide precursor and the polyimide of the present invention are used for an application to liquid crystal alignment film, alicyclic tetracarboxylic acids and their dianhydrides and their dicarboxylic diacid halides are preferred from the viewpoint of the transparency of the coating film, the electrical properties of the liquid crystal cells, etc. Particularly preferred are 1,2,3,4-cyclobutane tetracarboxylic dianhydride, 3,4-dicarboxy-1,2,3,4-tetrahydro-1-naphthalene succinic dianhydride, bicyclo[3,3,0]-octane-tetracarboxylic dianhydride, 3,5,6-tricarboxynorbornane-2:3,5:6 dianhydride, and 2,3,5-tricarboxy cyclopentyl acetic dianhydride. Further, these tetracarboxylic acids and their derivatives may be used alone or in combination as a mixture of two or more of them.

For the polyimide precursor and the polyimide of the present invention, the diamine component may be a copolymer of the diaminobenzene derivative represented by the formula (1) (hereinafter abbreviated as the diamine (1)) with a common diamine other than that (hereinafter referred to simply as a common diamine).

The common diamine to be used in such a case, is usually a primary diamine to be used for the synthesis of a polyimide precursor and a polyimide and is not particularly limited. Its specific examples include aromatic diamines such as p-phenylenediamine, m-phenylenediamine, 2,5-diaminotoluene, 2,6-diaminotoluene, 4,4'-diaminobiphenyl, 3,3'-dimethyl-4,4'-diaminobiphenyl, 3,3'-dimethoxy-4,4'-diaminobiphenyl, diaminodiphenyl methane, diaminodiphenyl ether, 2,2-diaminodiphenyl propane, bis(3,5-diethyl-4-aminophenyl) methane, diaminodiphenyl sulfone, diaminobenzophenone, diaminonaphthalene, 1,4-bis(4-aminophenoxy)benzene, 1,4-bis(4-aminophenyl)benzene, 9,10-bis (4-aminophenyl)anthracene, 1,3-bis(4-aminophenoxy)benzene, 4,4'-bis(4-aminophenoxy)diphenylsulfone, 2,2-bis [4-(4-aminophenoxy)phenyl]propane, 2,2-bis[4-(4-aminophenoxy)phenyl]hexafluoropropane, alicyclic diamines such as bis(4-aminocyclohexyl)methane and bis(4-amino-3-methylcyclohexyl)methane, and aliphatic diamines such as tetramethylenediamine and hexamethylenediamine, as well as diaminocyloxane shown by:

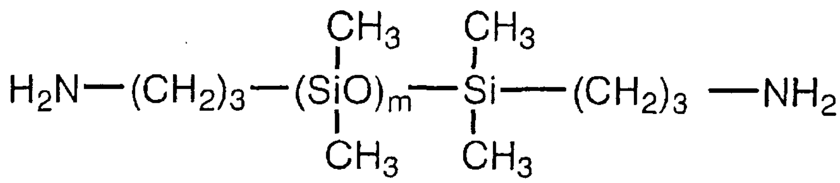

wherein m is an integer of from 1 to 10. Further, these diamines may be used alone or in combination as a mixture of two or more of them.

By adjusting the proportion of the mols of the diamine (1) to the total mols of the diamines to be used, at the time of obtaining the polyimide precursor and the polyimide of the present invention, it is possible to modify the surface properties of these polymers such as water repellency, and further, in a case where they are used as liquid crystal alignment films, it is possible to improve the wettability with liquid crystal and further to increase the pretilt angle of liquid crystal. The proportion of the mols of the diamine (1) to the total mols of diamines to be used in such a case, is at least 1 mol%.

Further, when they are used as liquid crystal alignment films, as the pretilt angle required in a usual liquid crystal display system (for example, a Twisted Nematic system, etc.), from about a few degrees to about ten degrees is used in many cases. Accordingly, the proportion of the mols of the diamine (1) to the total mol of diamines to be used is within a range of from 1 mol% to 49 mol%. Further, in the case of a vertical alignment system, the proportion of the mols of the diamine (1) is from 25 mol% to 100 mol%.

The polycondensation reaction of a tetracarboxylic acid or its derivative with the above diamine to obtain the polyimide precursor and the polyimide of the present invention, is not particularly limited, and a usual synthetic method for a polyimide can be employed. A method is common in which a tetracarboxylic dianhydride is used as a derivative of a tetracarboxylic acid, and it is reacted with a diamine in an organic polar solvent such as N-methylpyrrolidone or N,N'-dimethylacetoamide to obtain a polyamic acid as a polyimide precursor, followed by dehydration ring closure to obtain a polyimide. As the solvent for the reaction, in addition to the above, N,N'-dimethylformanide or γ-butylolactone may, for example, be used. Further, as the temperature during the synthesis of the polyimide precursor, an optional temperature of from -20°C to 150°C may be selected, but preferred is within a range of from -5°C to 100°C.

The ratio of the mols of the tetracarboxylic dianhydride to the mols of the diamine (the total mols of the diamine (1) and the common diamine) is preferably from 0.8 to 1.2. Like a usual polycondensation reaction, as this molar ratio becomes close to 1.0, the polymerization degree of the polymer to be formed, increases.

If the polymerization degree is too small, the strength of the polyimide film tends to be inadequate, and if the polymerization degree is too large, the operation efficiency at the time of formation of a polyimide film is likely to deteriorate. Accordingly, the polymerization degree of the product in this reaction is preferably adjusted to be from 10,000 to 1,000,000 by the weight average molecular weight as measured by gel permeation chromatography.

To convert the polyimide precursor to a polyimide, the dehydration ring closure may be carried out by heating the reaction solution of the polyimide precursor as it is at a temperature of from 100°C to 400°C, preferably from 120°C to 250°C, or by means of a catalyst such as pyridine/acetic anhydride. In such a case, it is of course preferred that the polyimide precursor is recovered and washed and then re-dissolved and converted to a polyimide.

<u>Treating agent for liquid crystal alignment</u>

The treating agent for liquid crystal alignment of the present invention is characterized in that it contains the polyimide precursor and/or the polyimide of the present invention, and its form is not particularly limited, but is usually a solution as dissolved in an organic solvent.

When the polyimide is to be used as a liquid crystal alignment film, it is necessary to form a polyimide film having a thickness of from 0.01 μm to 1.0 μm on a substrate. Usually, to form such a polyimide coating film, a method is employed wherein a polyimide precursor solution is coated on a substrate and heated on a substrate for imidation to form a polyimide coating film, or in a case where the polyimide is soluble in a solvent, a solution of the polyimide having the polyimide precursor preliminarily imidated, is coated on a substrate, followed by drying to form a polyimide coating film.

As the temperature for heating on the substrate for imidation, an optional temperature from 100°C to 400°C may be employed, but particularly preferred is within a range of from 150°C to 350°C. In the case of coating and drying the polyimide solution, it is sufficient if the solvent evaporates and is usually sufficient at a temperature of from 80°C to 150°C.

Coating on a substrate can be carried out by a printer for e.g. screen printing, offset printing or inkjet printing, as well as by a dip coater, a roll coater or a spinner. Especially in the case of a liquid crystal alignment film, the film thickness, the dimensional precision of the coating film and the uniformity of the surface will be particularly important, and therefore, it is common to employ a printing machine for coating the treating agent for liquid crystal alignment.

With respect to the polyimide precursor solution and the polyimide solution to be used for coating, the reaction solution may be used as it is or after dilution to a proper concentration, or it may be precipitated in a poor solvent such as water, methanol or ethanol, isolated and washed and then re-dissolved in a solvent for use.

The solvent to be used for dilution or redissolution is not particularly limited so long as it is capable of dissolving the polyimide precursor or the polyimide. For example, 2-pyrrolidone, N-methylpyrrolidone, N-ethylpyrrolidone, N,N-dimethylacetoamide, N,N-dimethylformamide or γ-butylolactone may be mentioned. These solvents may be used alone or in combination as a mixture.

Further, even a solvent which is incapable of providing a uniform solution of the polyimide precursor or the polyimide by itself, may be added to the coating solution within a range where a uniform solution can be obtained. As such an example, ethyl cellosolve, butyl cellosolve, ethyl carbitol, butyl carbitol, ethyl carbitol acetate or ethylene glycol may be mentioned. These solvents may be used alone or in combination as a mixture.

It is also preferred to add to the coating solution e.g. a coupling agent for the purpose of improving the adhesion of the polyimide film to the substrate, or a dielectric or electroconductive substance for the purpose of changing electrical properties such as the dielectric constant and electroconductivity of the polyimide film.

The treating agent for liquid crystal alignment of the present invention can be used also as mixed with other resin component not containing the diaminobenzene derivative represented by the formula (1) in its structure. To mix such a resin component, it is simple to form a mixed solution at the time of preparing the coating solution.

The treating agent for liquid crystal alignment of the present invention may be formed into a uniform film on a substrate, followed by treatment for alignment by rubbing or light irradiation, or no treatment for alignment, and used as a liquid crystal alignment film.

Now, the present invention will be described in further detail with reference to Examples, but the present invention is by no means restricted thereto.

EXAMPLE 1

Synthesis of diamine (5)

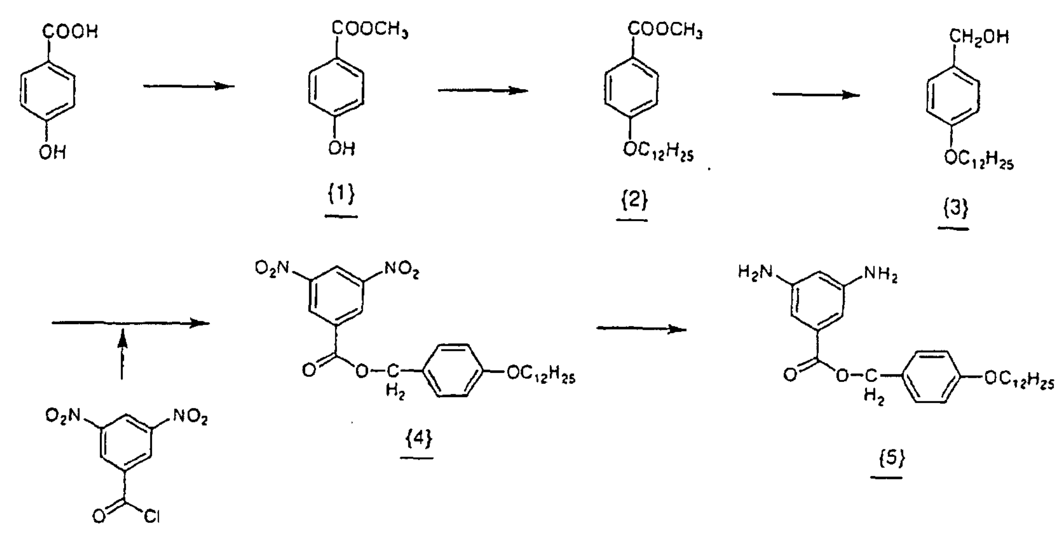

{1}

{2}

{3}

{4}

{5}

Into a 1,000 ml eggplant type flask, 4-hydroxy benzoic acid (50 g), concentrated sulfuric acid (50 ml) and

methanol (500 ml) were added, and refluxed and stirred for 8 hours. After completion of the reaction, the reaction solution was distilled off under reduced pressure. The residue was washed with water to obtain colorless crystal {1} (46.4 g, 84%, mp: 130-131°C).

$^1$H-NMR(d-DMSO, δ ppm) : 10.35(1H,S), 7.81(2H,d), 6.85(2H,d), 3.79(3H,S).

Into a 500 ml three necked flask, {1} (20.08 g), 1-bromododecane (36.07 g), potassium carbonate (20.01 g) and DMF (300 ml) were added, followed by stirring at 100°C for 7 hours. After completion of the reaction, the reaction solution was filtered while it was hot. The filtrate was left to stand at room temperature, and precipitated solid was collected by filtration to obtain colorless crystals {2} (34.67 g, 82%, mp: 58-59°C).

$^1$H-NMR ($CDCl_3$, δ ppm) : 7.98(2H,d), 6.90(2H,d), 4.00 (2H, t), 3.88(3H,S), 1.80(2H,m), 1.46(2H,m), 1.27(16H, broad), 0.88(3H,t).

In a nitrogen atmosphere, into a 500 ml four necked flask, $LiAlH_4$ (2.25 g) and THF (150 ml) were added to prepare a suspension of $LiAlH_4$. A THF solution (150 ml) of {2} (15.76 g) was dropwise added thereinto. After completion of the dropwise addition, refluxing and stirring were carried out for 28 hours. After completion of the reaction, water was dropwise added to the reaction solution under cooling with ice, and then, 1N-HCl was added. The oily product was distilled off under reduced pressure, and the obtained solid was recrystallized from acetonitrile to obtain colorless crystals {3} (11.21 g, 75%, mp: 51-52°C).

$^1$H-NMR (d-DMSO, δ ppm) : 7.20(2H,d), 6.85(2H,d), 5.02(1H,t), 4.40(2H,d), 3.92(2H,t), 1.68(2H,m), 1.38(2H, m), 1.24(16H,broad), 0.85(3H,t).

Into a 500 ml four necked flask, {3} (25.08 g), triethylamine (8.80 g) and THF (150 ml) were added. Then, a THF solution (100 ml) of 3,5-dinitrobenzoyl chloride (19.94 g) was dropwise added thereto. After completion of the dropwise addition, refluxing and stirring were carried out for 3 hours. The reaction solution was distilled off under reduced pressure and extracted by means of a chloroform, a 1N NaOH solution and water. The organic layer was distilled off under reduced pressure to obtain slightly yellow crystals {4} (19.36 g, 47%, mp: 53-56°C).

$^1$H-NMR(d-DMSO, δ ppm) : 9.03(1H,S), 8.89(2H,S), 7.45(2H,d), 6.96(2H,d), 5.39(2H,S), 3.97(2H,t), 1.67(2H, m), 1.40(2H,m), 1.24(16H,broad), 0.85(3H,t).

Into a 500 ml four necked flask, {4} (20.01 g) and 1,4-dioxane (300 ml) were added, and the reactor was flushed with nitrogen, and then $PtO_2$ (1.00 g) was added. Thereafter, the interior of the reactor was adjusted to be a hydrogen atmosphere, and stirring was carried out at 60°C for 20 hours and at room temperature for 28 hours. After completion of the reaction, $PtO_2$ was removed by filtration, and the filtrate was distilled off under reduced pressure. The residue was recrystallized from n-hexane to obtain slightly yellow crystals {5} (15.54 g, 88%, mp: 52-53°C).

$^1$H-NMR(d-DMSO, δ ppm) : 1 7.35(2H,d), 6.89 (2H, d), 6.79(2H,s), 6.17 (1H, s), 5,23 (2H, s), 3.95 (2H, t), 1.78 (2H,m) , 1.43 (2H,m) , 1.30 (16H, broad), 0.88 (3H, t).

EXAMPLE 2

Synthesis of diamine {7}

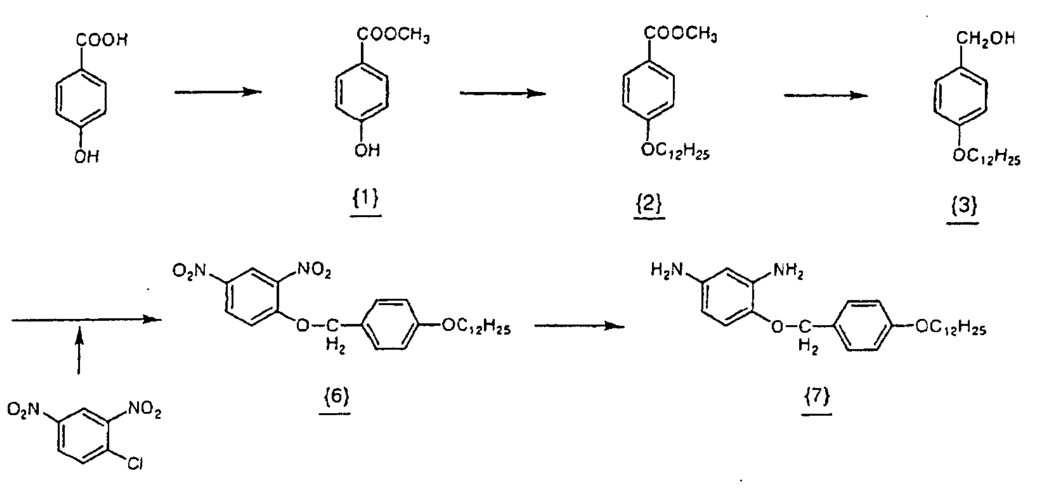

{1} {2} {3} {6} {7}

Into a 500 ml four necked flask, {3} (17.00 g), 2,4-dinitrochlorobenzene (11.78 ml), potassium carbonate (18.09 g), 18-crown ether (5.38 g) and THF (250 ml) were added, and refluxed and stirred for 18 hours. After completion of

the reaction, the reaction solution was distilled off under reduced pressure, and methanol was added to the residue, whereupon precipitated solid was collected by filtration to obtain slightly brown crystals {6} (12.01 g, 45%, 54-55°C).

$^1$H-NMR (d-DMSO, δ ppm) : 8.91 (1H, s), 8.51 (1H, d), 8.08 (1H, d), 7.20 (2H, d), 6.85(2H,d), 4.40(2H,S), 3.92 (2H,t), 1.68(2H,m), 1.39 (2H, m), 1.24(16H,broad), 0.85(3H,t).

Into a 500 ml four necked flask, {6} (12.01 g) and 1,4-dioxane (170 ml) were added. The reactor was flushed with nitrogen, and then, $PtO_2$ (1.11 g) was added. Then, the interior of the reactor was changed to a hydrogen atmosphere, followed by stirring at 45°C for 39 hours and at room temperature for 197 hours. After completion of the reaction, $PtO_2$ was removed by filtration, and the filtrate was distilled off under reduced pressure. The residue was purified by silica gel column chromatography (ethyl acetate/n-hexane=6/4) to obtain slightly yellow crystals {7} (2.50 g, 24%, mp: 52-53°C).

$^1$H-NMR($CDCl_3$, δ ppm) : 7.32(2H,d), 6.88(2H,d), 6.68 (1H, s), 6.13(1H,s) 6.04(1H,s), 4.89 (2H, S), 3.95(2H,t), 3.71(2H,broad), 3.33(2H,broad), 1.77 (2H, m), 1.45(2H,m), 1.26(16H,broad), 0.88(3H,t).

EXAMPLE 3

Production of polyimide

PREPARATION EXAMPLE 1

Using the diamine {5} (1.64 g, 3.75 mmol) obtained in Example 1, 2,2'-bis[4-(4-aminophenoxy)phenyl]propane (0.51 g, 1.25 mmol), 1,2,3,4-cyclobutane tetracarboxylic dianhydride (0.98 g, 5.00 mmol) and N-methylpyrrolidone (17.50 g), stirring was carried out at room temperature to carry out a polycondensation reaction to obtain a polyimide precursor solution A having a solid content concentration of 14 wt%. The viscosity of this solution was 100 MPa·s (25°C; by E model viscometer), and the weight average molecular weight as measured by GPC (Gel Permeation Chromatography) was 48,900.

PREPARATION EXAMPLE 2

Using the diamine {5} (1.10 g, 2.50 mmol) obtained in Example 1, 2,2'-bis[4-(4-aminophenoxy)phenyl]propane (1.03 g, 2.50 mmol), 1,2,3,4-cyclobutane tetracarboxylic dianhydride (0.98 g, 5.00 mmol) and N-methylpyrrolidone (17.31 g), stirring was carried out at room temperature to carry out a polycondensation reaction to obtain a polyimide precursor solution B having a solid content concentration of 15 wt%. The viscosity of this solution was 128 MPa·s (25°C; by E model viscometer), and the weight average molecular weight as measured by GPC was 38,800.

PREPARATION EXAMPLE 3

Using the diamine {5} (0.54 g, 1.25 mmol) obtained in Example 1, 2,2'-bis[4-(4-aminophenoxy)phenyl]propane (1.54 g, 3.75 mmol), 1,2,3,4-cyclobutane tetracarboxylic dianhydride (0.98 g, 5.00 mmol) and N-methylpyrrolidone (17.11 g), stirring was carried out at room temperature to carry out a polycondensation reaction to obtain a polyimide precursor solution C having a solid content concentration of 15 wt%. The viscosity of this solution was 527 MPa·s (25°C; by E model viscometer), and the weight average molecular weight as measured by GPC was 90,000.

PREPARATION EXAMPLE 4

Using the diamine {5} (0.33 g, 0.75 mmol) obtained in Example 1, 2,2'-bis[4-(4-aminophenoxy)phenyl]propane (1.74 g, 4.25 mmol), 1,2,3,4-cyclobutane tetracarboxylic dianhydride (0.98 g, 5.00 mmol) and N-methylpyrrolidone (17.03 g), stirring was carried out at room temperature to carry out a polycondensation reaction to obtain a polyimide precursor solution D having a solid content concentration of 15 wt%. The viscosity of this solution was 1,280 MPa·s (25°C; by E model viscometer), and the weight average molecular weight as measured by GPC was 129,000.

PREPARATION EXAMPLE 5

Using the diamine {7} (1.00 g, 2.50 mmol) obtained in Example 2, 2,2'-bis[4-(4-aminophenoxy)phenyl]propane (1.03 g, 2.50 mmol), 1,2,3,4-cyclobutane tetracarboxylic dianhydride (0.98 g, 5.00 mmol) and N-methylpyrrolidone (16.75 g), stirring was carried out at room temperature to carry out a polycondensation reaction to obtain a polyimide precursor solution E having a solid content concentration of 15 wt%. The viscosity of this solution was 156 MPa·s (25°C; by E model viscometer), and the weight average molecular weight as measured by GPC was 39,800.

PREPARATION EXAMPLE 6

Using the diamine {7} (0.30 g, 0.75 mmol) obtained in Example 2, 2,2'-bis[4-(4-aminophenoxy)phenyl]propane (1.74 g, 4.25 mmol), 1,2,3,4-cyclobutane tetracarboxylic dianhydride (0.98 g, 5.00 mmol) and N-methylpyrrolidone (16.86 g), stirring was carried out at room temperature to carry out a polycondensation reaction to obtain a polyimide precursor solution F having a solid content concentration of 15 wt%. The viscosity of this solution was 344 MPa·s (25°C; by E model viscometer), and the weight average molecular weight as measured by GPC was 615,000.

EXAMPLES 4 to 9

Production of treating agents for liquid crystal alignment

The polyimide precursor solutions (A to F) obtained in Example 3 (Preparation Examples 1 to 6) were diluted with N-methylpyrrolidone (NMP) or γ-butylolactone (γ-BL) to prepare treating agents for liquid crystal alignment. The results are shown in Table 1.

EXAMPLES 10 to 15

Production of liquid crystal alignment films

The treating agents for liquid crystal alignment obtained in Examples 4 to 9 were spin-coated on glass substrates and thermally treated at 180°C or 250°C to form polyimide coating films. And, the water repellency of the polyimide surface, and the uniformity in alignment and the pretilt angle of liquid crystal when formed into a liquid crystal alignment film, were measured by the following methods. Evaluation of the water repellency was carried out by the following method. The polyimide precursor solution or the polyimide solution was diluted with N-methylpyrrolidone or γ-butylolactone to obtain a solution having a resin concentration of from 3 to 10 wt%. This solution was spin-coated on a glass substrate having transparent electrodes and thermally treated at 80°C for 10 minutes and at 180°C or 250°C for one hour to form a uniform polyimide coating film. The contact angles of water and methylene iodide on this coating film were measured, and the surface energy was calculated by the following formula.

$$(1+\cos\theta)\times\gamma_L=2(\gamma_s^{d}\times\gamma_L^{P})^{1/2}+2(\gamma_s^{d}\times\gamma_L^{P})^{1/2}$$

$$\gamma_L=\gamma_L^{d}+\gamma_L^{p}$$

$$\gamma_s=\gamma_s^{d}+\gamma_s^{p}$$

θ: The contact angle of the liquid on the coating film
$\gamma_L$: The surface tension of the liquid
$\gamma_L^d$: Dispersion term of the surface tension of the liquid
$\gamma_L^p$: Polarity term of the surface tension of the liquid
$\gamma_s$: The surface tension of the coating film
$\gamma_s^d$: Dispersion term of the surface tension of the coating film
$\gamma_s^p$: Polarity term of the surface tension of the coating film

Here, by substituting the contact angle of water as $\theta_1$, the contact angle of methylene iodide as $\theta_2$, the surface tension of water ($\gamma_L$=72.8, $\gamma_L^d$=21.8, $\gamma_L^p$=51.0) (dyn/cm) and the surface tension of methylene iodide ($\gamma_L$=50.8, $\gamma_L^d$=49.5, $\gamma_L^p$=1.3) (dyn/cm) , $\gamma_s^d$ and $\gamma_s^p$ were obtained from:

$$(1+\cos\theta_1)\times 72.8=2(\gamma_s^{d}\times 21.8)^{1/2}+2(\gamma_s^{d}\times 51.0)^{1/2}$$

$$(1+\cos\theta_2)\times 50.8=2(\gamma_s^{d}\times 49.5)^{1/2}+2(\gamma_s^{d}\times 1.3)^{1/2}$$

and the surface energy of the polyimide coating film was calculated from $\gamma_s=\gamma_s^d+\gamma_s^p$.

Further, the measurements of the uniformity in alignment and the pretilt angle of liquid crystal when formed

into a liquid crystal alignment film, were carried out by the following methods. The measurement of the pretilt angle was carried out as follows. The polyimide precursor or the polyimide solution was diluted with N-methylpyrrolidone or γ-butylolactone to obtain a solution having a resin concentration of from 3 to 10 wt%. This solution was spin-coated on a glass substrate having a transparent electrodes and thermally treated at 80°C for 10 minutes and at 180°C or 250°C for one hour to form a uniform polyimide coating film. This coating film was rubbed with a cloth, and then, a pair of such films were assembled with a spacer sandwiched therebetween so that the rubbing directions were in parallel, and liquid crystal (ZLI-2293, manufactured by Merck Company) was injected to prepare a cell having a homeotropic or homogenous alignment. With respect to this cell, after thermal treatment at 95°C for 5 minutes, the uniformity in alignment of liquid crystal was confirmed by a polarizing microscope, and with respect to one thermally treated at 120°C for one hour, the pretilt angle was measured by a crystal rotation method or a magnetic field capacity method.

The results are shown in Tables 1 and 2. Further, for the purpose of comparison, the following diamine {9} was synthesized, a polyimide precursor was prepared, a liquid crystal alignment film was prepared, and evaluation was carried out. The results are also shown in Tables 1 and 2.

COMPARATIVE EXAMPLE 1

Synthesis of diamine {9}

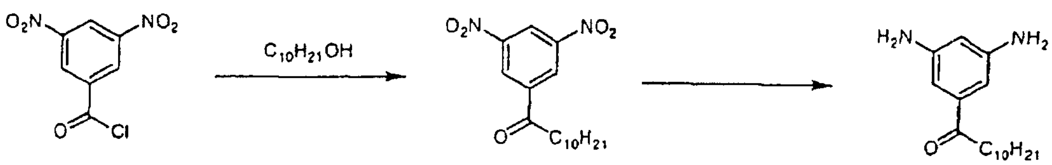


{8} {9}

Into a 500 ml four necked flask, n-decyl alcohol (12.36 g), triethylamine (7.94 g) and THF (200 ml) were added. A THF solution (150 ml) of 3,5-dinitrobenzoyl chloride (15.41 g) was dropwise added thereto. After completion of dropwise addition, refluxing and stirring were carried out for three hours. The reaction solution was distilled off under reduced pressure and extracted by means of chloroform, a 1N NaOH solution and water. The organic layer was distilled off under reduced pressure to obtain slightly yellow crystals {8} (21.46 g, 94%).

$^1$H-NMR (d-DMSO, δ ppm) : 9.04 (1H, S), 8.90(2H,S), 4.40(2H,t), 1.77(2H,m), 1.40-1.18 (14H, broad), 0.85 (3H, t).

Into a 500 ml four necked flask, {8} (15.00 g) and 1,4-dioxane (250 ml) were added, the reactor was flushed with nitrogen, and then, Pd-C (1.50 g) was added. Then, the interior of the reactor was changed to a hydrogen atmosphere, followed by stirring at room temperature for 36 hours. After completion of the reaction, Pd-C was removed by filtration, and the filtrate was distilled off under reduced pressure. The residue was recrystallized from n-hexane to obtain slightly yellow crystals {9} (1.68 g, 76%).

$^1$H-NMR($CDCl_3$, δ ppm) : 6.78(2H,s), 6.19(2H,s), 4.25(2H,t), 3.60(4H,broad), 1.73(2H,m), 1.45(2H,m), 1.27(12H, broad), 0.88(3H,t).

COMPARATIVE EXAMPLE 2

Production of polyimides

PREPARATION EXAMPLE 7

Using the diamine {9) (1.10 g, 3.75 mmol) obtained in Comparative Example 1, 2,2'-bis[4-(4-aminophenoxy) phenyl]propane (0.51 g, 1.25 mmol), 1,2,3,4-cyclobutane tetracarboxylic dianhydride (0.98 g, 5.00 mmol) and N-methylpyrrolidone (14.40 g), stirring was carried out at room temperature to carry out a polycondensation reaction to obtain a polyimide precursor solution G having a solid content concentration of 13 wt%. The viscosity of this solution was 54 MPa·s (25°C; by E model viscometer), and the weight average molecular weight as measured by GPC was 14,700.

PREPARATION EXAMPLE 8

Using the diamine {9} (0.73 g, 2.50 mmol) obtained in Comparative Example 1, 2,2'-bis [4-(4-aminophenoxy) phenyl]propane (1.03 g, 2.50 mmol), 1,2,3,4-cyclobutane tetracarboxylic dianhydride (0.98 g, 5.00 mmol) and N-methylpyrrolidone (15.24 g), stirring was carried out at room temperature to carry out a polycondensation reaction to obtain a polyimide precursor solution H having a solid content concentration of 14 wt%. The viscosity of this solution was 435 MPa·s (25°C; by E model viscometer), and the weight average molecular weight as measured by GPC was 34,800.

PREPARATION EXAMPLE 9

Using the diamine {9} (0.73 g, 3.75 mmol) obtained in Comparative Example 1, 2,2'-bis[4-(4-aminophenoxy) phenyl]propane (4.62 g, 11.25 mmol), 1,2,3,4-cyclobutane tetracarboxylic dianhydride (2.94 g, 15.00 mmol) and N-methylpyrrolidone (48.72 g), stirring was carried out at room temperature to carry out a polycondensation reaction to obtain a polyimide precursor solution I having a solid content concentration of 15 wt%. The viscosity of this solution was 536 MPa·s (25°C; by E model viscometer), and the weight average molecular weight as measured by GPC was 61,400.

COMPARATIVE EXAMPLES 3 to 5

Production of treating agents for liquid crystal alignment

Using the polyimide precursor solutions (G to I) obtained in Comparative Example 2 (Preparation Examples 7 to 9), treating agents for liquid crystal alignment were produced by the same method as in Examples 4 to 9. COMPARATIVE EXAMPLES 6 to 8

Production of liquid crystal alignment films

Using the treating agents for liquid crystal alignment obtained in Comparative Examples 3 to 5, liquid crystal alignment films were produced by the same method as in Examples 10 to 15, and the water repellency on the polyimide surface and the uniformity in alignment and the pretilt angle of liquid crystal when formed into a liquid crystal alignment film, were measured.

INDUSTRIAL APPLICABILITY

The diaminobenzene derivative of the present invention can easily be synthesized, and by using such a derivative as a part of the material, it is possible to obtain a polyimide precursor and a polyimide having the surface properties such as water repellency improved with a high molecular weight. Further, a liquid crystal alignment film prepared by using such a treating agent for liquid crystal alignment containing such a polymer has a high effect to increase the pretilt angle and is excellent in the thermal stability of the pretilt angle.

Table 1

| Production of treating agents for liquid crystal alignment | | | | |
|---|---|---|---|---|
| | Polyimide precursor solution | Solvent for dilution (NMP: γ-BL) | Treating agent for liquid crystal alignment | |
| | | | Viscosity (m·Pa·S) | Solid content concentration |
| Example | | | | |
| 4 | A | 8:2 | 19.5 | 6.93 |
| 5 | B | 8:2 | 21.6 | 7.03 |
| 6 | C | 8:2 | 18.3 | 4.53 |
| 7 | D | 8:2 | 23.9 | 3.90 |
| 8 | E | 8:2 | 28.9 | 6.01 |
| 9 | F | 8:2 | 43.5 | 4.49 |

Table 1 (continued)

| Production of treating agents for liquid crystal alignment | | | | |
|---|---|---|---|---|
| Comparative Example | Polyimide precursor solution | Solvent for dilution (NMP: γ-BL) | Treating agent for liquid crystal alignment | |
| | | | | |
| 3 | G | 8:2 | 15.5 | 7.02 |
| 4 | H | 8:2 | 17.7 | 4.53 |
| 5 | I | 8:2 | 14.2 | 4.04 |

Table 2

| Results of evaluation of the water repellency of coating films | | | | | |
|---|---|---|---|---|---|
| | Treating agent for liquid crystal alignment | Diamine (amount %) | Contact angle of liquid (°) | | |
| | | | Water | Methylene iodide | Surface energy |
| | Example | | | | |
| Example 10 | 4 | {5}(75) | 91.7 | 53.6 | 32.4 |
| | | | 89.3 | 49.9 | 34.6 |
| 11 | 5 | {5}(50) | 89.4 | 51.8 | 33.6 |
| | | | 89.6 | 49.6 | 34.7 |
| 12 | 6 | {5}(25) | 81.9 | 46.2 | 37.5 |
| | | | 82.4 | 42.7 | 39.0 |
| 13 | 7 | {5}(15) | 80.2 | 42.3 | 39.7 |
| | | | 73.8 | 40.5 | 42.1 |
| 14 | 8 | {7}(50) | 91.6 | 50.5 | 35.4 |
| | | | 87.3 | 48.0 | 36.0 |
| 15 | 9 | {7}(15) | 77.4 | 39.1 | 40.1 |
| | | | 78.1 | 34.9 | 42.2 |
| | Comparative Example | | | | |
| Comparative Example 6 | 3 | {9}(75) | 74.5 | 49.6 | 38.3 |
| | | | 75.4 | 44.7 | 40.0 |
| 7 | 4 | {9}(50) | 70.3 | 45.1 | 41.7 |
| | | | 77.6 | 42.1 | 40.4 |
| 8 | 5 | {9}(25) | 81.6 | 39.7 | 40.5 |
| | | | 76.1 | 38.3 | 42.3 |
| * The upper represents film formation by thermal treatment at 180°C for one hour, and the lower represents film formation by thermal treatment at 250°C for one hour. | | | | | |

Table 3

| Results of evaluation of the pretilt angle | | | | |
|---|---|---|---|---|
| | Treating agent for liquid crystal alignment | | | Pretilt angle (°) |
| | | Diamine (amount %) | Pretilt angle (°) | After treatment at 120°C for one hour |
| | Example | | | |
| Example 10 | 4 | {5}(75) | 89.8 | 89.8 |
| | | | 89.1 | 89.4 |
| 11 | 5 | {5}(50) | 89.4 | 89.7 |
| | | | 88.6 | 88.9 |
| 12 | 6 | {5}(25) | 69.4 | 41.4 |
| | | | 34.2 | 12.5 |
| 13 | 7 | {5}(15) | 3.2 | 3.3 |
| | | | 4.8 | 5.9 |
| 14 | 8 | {7}(50) | 89.7 | 89.0 |
| | | | 72.0 | 66.9 |
| 15 | 9 | {7}(15) | 3.9 | 0.7 |
| | | | 6.1 | 6.5 |
| | Comparative Example | | | |
| Comparative Example 6 | 3 | {9}(75) | 79.7 | 77.3 |
| | | | --- | --- |
| 7 | 4 | {9}(50) | 9.0 | 4.4 |
| | | | 14.4 | 8.4 |
| 8 | 5 | {9}(25) | 3.8 | 3.2 |
| | | | 5.6 | 6.3 |
| * The upper represents film formation by thermal treatment at 180°C for one hour, and the lower represents film formation by thermal treatment at 250°C for one hour. * In each cell, uniform alignment without any defect was observed. | | | | |

**Claims**

**1.** A diaminobenzene derivative represented by the formula (1):

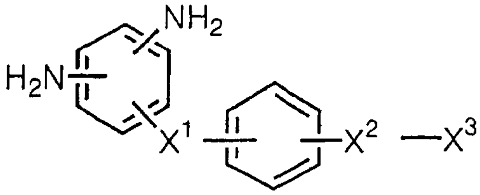

(1)

wherein $X^1$ is a bivalent organic group selected from $-OCH_2-$, $-CH_2O-$, $-COOCH_2-$ and $-CH_2OOC-$, $X^2$ is -O-, and

$X^3$ is an alkyl or fluoroalkyl group having from 1 to 22 carbon atoms, or a cyclic substituent selected from aromatic rings, aliphatic rings, heterocyclic rings and their substituted groups.

**2.** A polyimide precursor and a polyimide synthesized by using the diaminobenzene derivative represented by the formula (1) as defined in Claim 1, as a part of the material.

**3.** A treating agent for liquid crystal alignment containing the polyimide precursor and/or the polyimide as defined in Claim 2.

**INTERNATIONAL SEARCH REPORT**

International application No.
PCT/JP01/11487

A. CLASSIFICATION OF SUBJECT MATTER
Int.Cl$^7$ C08G73/10, G02F1/1337, C09K19/56, C07C217/86, C07C229/60

According to International Patent Classification (IPC) or to both national classification and IPC

B. FIELDS SEARCHED

Minimum documentation searched (classification system followed by classification symbols)
Int.Cl$^7$ C08G73/00-73/26

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| X | JP 4-335615 A (Fuji Photo Film Co., Ltd.), 24 November, 1992 (24.11.1992), Claims (Family: none) | 1-3 |
| X | JP 3-121132 A (Japan Carlit Co., Ltd.), 23 May, 1991 (23.05.1991), Claims; page 7, Compound Nos. 58, 61 (Family: none) | 1-3 |
| A | EP 682283 A1 (NISSAN CHEMICAL INDUSTRIES LTD.), 15 November, 1995 (15.11.1995), Full text & US 5608033 A & JP 7-301805 A & JP 7-301806 A | 1-3 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 January, 2002 (23.01.02) | 05 February, 2002 (05.02.02) |
| Name and mailing address of the ISA/ Japanese Patent Office | Authorized officer |
| Facsimile No. | Telephone No. |

Form PCT/ISA/210 (second sheet) (July 1992)